# EUROPEAN PATENT SPECIFICATION

(11) **EP 0 801 938 B1**
(45) Date of publication and mention of the grant of the patent: **23.06.2004**
(21) Application number: 97106282.3
(22) Date of filing: 16.04.1997
(51) Int. Cl.: A61F 7/12

(54) **Thermal treatment apparatus**
Vorrichtung zur Wärmebehandlung
Appareil de traitement thermique

(30) Priority: 16.04.1996 US 632830
(43) Date of publication of application: 22.10.1997
(73) Proprietor: Wit IP Corporation, Southborough, MA 01772 (US)
(72) Inventor: Eshel, Uzi, Herzlia (IL); Crabtree, Graham J., Herzlia (IL); Lazarovitz, Jacob, Hod Hasharon (IL)
(74) Representative: von Samson-Himmelstjerna, Friedrich R., Dipl.-Phys.

(56) References cited:
- EP-A- 0 672 401
- WO-A-94/10948
- US-A- 4 860 744
- US-A- 5 151 100
- US-A- 5 257 977
- US-A- 5 281 213
- US-A- 5 431 648

## Description

The present invention relates to a thermal treatment apparatus which is able to provide a desired temperature to a minimal quantity of fluid circulating in a closed system of the thermal treatment apparatus.

The thermal treatment apparatus can be used to selectively treat a targeted tissue by applying fluid-induced thermal therapy, as disclosed in US-A-5 257 977 and EP-A-0 672 401.

Fluid induced thermal therapy is based on localized heating of a targeted tissue to a predetermined temperature by means of circulating fluid, while keeping the temperature of adjacent healthy tissues unchanged. Relative to therapy methods which use laser, microwave, radiofrequency and ultrasound to produce heat within a tissue, the fluid-induced thermotherapy allows better control of the temperature and the limits of the treated site.

Alternatively, a thermal treatment apparatus according to the present invention may be used for selectively preserving a portion of a tissue treated by cryosurgery, by selectively heating that portion of the tissue.

Various attempts have been made to provide devices for heating an amount of fluid circulating in a closed systems such as fluid-induced thermal therapy systems. Prior art includes heating bath circulators and a heater based on a container serving as a water reservoir. An example is described in EP-A-0 672 401, according to which the preamble of claim 1 has been drafted.

However, such configurations consume a considerable amount of energy, since a large quantity of fluid has to be heated (or cooled). Moreover, a considerable damage may be caused in the event of system's failure, by the leakage of a large quantity of fluid into the body cavity.

Is therefore an object of the present invention to provide a thermal treatment apparatus which enables thermal treatments to be carried out in a more efficient and safer way.

### SUMMARY OF THE INVENTION

According to the present invention there is provided a thermal treatment apparatus for the treatment of the human body comprising a temperature-setting device for providing a predetermined temperature to a quantity of fluid circulating in a closed-loop system of the thermal treatment apparatus, configured to heat and/or cool the circulating fluid to a predetermined temperature, comprising a tubular thermal element having a body that encloses a fluid flow passage and an axial length with opposing first and second ends, said tubular thermal element having thermal conductivity and being sized and configured to allow circulating fluid to flow therethrough, wherein, in operation, said tubular thermal element body is configured to be heated and/or cooled and to transfer its thermal energy to heat and/or cool the circulating fluid to a predetermined temperature as the circulating fluid flows therethrough.

Preferably, the temperature setting device has a housing element for receiving and anchoring said tubular element therein, and for electrically connecting the tubular element to an electrical circuit; and a transformer element electrically connectable to the electrical circuit.

According to further features in preferred embodiments of the invention described below, the tubular element has two ends connectable to electrically and thermally insulating tubular elements.

Further, the housing element includes fastening elements, comprising: a connector member having a relatively good electrical conductivity, connectable to an electrical circuit; and fastening member for anchoring the tubular member between the fastening member and the connector member.

According to still further features in the described preferred embodiments, the temperature-setting device may include a sensor for sensing the temperature of the tubular element.

The sensor and the transformer element may be electrically connected to a controller. In such configuration, the controller may control the duty cycle of the transformer according to the temperature sensed by the sensor. Alternatively, the controller may interrupt the operation of the transformer and the electrical circuit upon over--heating of the tubular element.

The tubular element and the insulating tubular elements may be disposable.

According to another embodiment, the housing element comprises a thermal conducting member disposed within a bath of a cooling substance, the thermal conducting member featuring a recess for receiving the tubular element therein. The recess preferably defines a lowered area relative to the surface of the cooling substance, for trapping cold air. The recess may be used for receiving a small quantity of fluid for improving the thermal coupling between the thermal conducting member and the tubular element.

According to further features of this embodiment, the thermal conducting member features extensions for increasing the area of contact between the thermal conducting member and the cooling substance.

The tubular element may alternately be placed in embodiments functioning as heaters and embodiments functioning as coolers, thereby alternately heating and cooling the fluid circulating therethrough.

According to yet another embodiment, the housing element is in the form of two thermal conducting plates, each of which controllably connectable to a heating and/or cooling source such as a peltier heat pump element.

Each of the thermal conducting plates may feature a recess, the recesses defining a tunnel for receiving the tubular element therein, preferably under pressure.

The thermal conducting plates may alternately heat and cool the tubular element, thereby alternately heating and cooling the fluid circulating therethrough.

In a preferred embodiment, the thermal treatment apparatus further comprises:(a) a catheter adapted for insertion into the human body, said catheter including a thermal treatment section for thermally treating targeted tissue in the human body by exposing targeted tissue to circulating fluid heated to a predetermined temperature that is contained in said catheter thermal treatment section; (b) the temperature-setting being in fluid communication with said catheter such that the circulating fluid flows through said tubular heating element as it travels in the circulating flow path; (c) a pump operably associated with said tubular heating element and catheter, said pump configured to drive said fluid through said circulating flow path; (d) a power source operably associated with said pump and said tubular heating element; and (e) a controller operably associated with said tubular heating element (3) and said power source, wherein, in operation, said tubular heating element body is controllably heated to thereby heat the circulating fluid traveling therethrough to a predetermined temperature.

According to further features in preferred embodiments of the invention described below, the thermal treatment apparatus may further include a second thermal sensor assembly for sensing the temperature of fluid circulating out of the catheter.

According to still further features in preferred embodiments of the invention , the thermal treatment apparatus may include an air trapping element, for trapping air circulating in the system.

The present invention successfully addresses the shortcomings of the presently known configurations by providing a temperature-setting device for providing a predetermined temperature to a minimal quantity of fluid circulating in a closed system.

The present invention discloses a novel temperature-setting device which allows to alternately heat and cool a component of such closed system, the component being in the form of a tubular element having relatively good thermal conductivity, by alternately placing the component in embodiments of the device functioning as heaters and embodiments functioning as coolers.

A technique for selectively thermally treating a targeted tissue adjacent a subject's body cavity comprises the steps of: (a) inserting into a subject's body cavity a catheter, including a thermal treating section for thermally treating said targeted tissue adjacent to the body cavity by circulating fluid of a predetermined temperature through said thermal treating section, the catheter being disposable; (b) heating fluid circulating in a closed system including the catheter, and further including: a pump for pumping fluid through the closed system; thermal sensor assemblies for sensing the temperature of fluid circulating into and out of the catheter; and a temperature-setting device for heating the fluid circulating therethrough, the temperature-setting device including: (i) a tubular element having relatively low electrical conductivity and good thermal conductivity for receiving a quantity of circulating fluid, the tubular element having two ends connectable to electrically and thermally insulating tubular elements, the tubular element and the insulating tubular elements being disposable; (ii) a housing element for receiving the tubular element, the housing element including fastening elements, including: a connector member having relatively good electrical conductivity, the connector member connectable to an electrical circuit; and a fastening member for anchoring the tubular element between the fastening member and the connector member; (iii) a transformer element connectable to the electrical circuit, the transformer element electrically connected to the controller.

The catheter may include a proximal end formed with an inflatable thermal treating section to be located within the body cavity, and a distal end to be located externally of the body cavity; the catheter being formed with first and second passageways extending from the distal end to the inflatable thermal treating section for circulating fluid of a predetermined temperature through the inflatable thermal treating section.

The catheter may include a proximal end formed with an inflatable anchoring section for anchoring the catheter in the body cavity, a distal end to be located externally of the body cavity, and an inflatable thermal treating section adjacent the proximal end to be located near the targeted tissue; the catheter being formed with first and second passageways extending from the distal end to the inflatable thermal treating section for circulating fluid of a predetermined temperature through the inflatable thermal treating section, and a third passageway extending from the distal end to the inflatable anchoring section for introducing unheated fluid into the inflatable anchoring section.

The catheter may further include thermal insulation surrounding the first and second passageways from close to the distal end to close to the inflatable thermal section, the thermal insulation including a plurality of separate compartments containing a non-heated fluid, the compartments extending axially along the catheter, thereby the inflatable thermal treating section and the targeted tissue in its proximity may be heated to a desired temperature without correspondingly heating non-targeted tissues.

The tubular element of the temperature-setting device, the insulating tubular elements and other tubular elements constituting the closed system may be disposable.

The pump may include: (a) a housing formed with a substantially cylindrical cavity having an inner surface, the housing provided with a lid, the lid having a depending skirt removably engageable within the cylindrical cavity so as to form an inset lining around a part of the inner surface; (b) a rotor rotatably mounted within the cylindrical cavity, the rotor carrying rollers; and (c) a peristaltic tube, the peristaltic tube being disposable, such that, when the depending skirt is not engaged in the cylindrical cavity, the peristaltic tube may be easily inserted between the rollers and the inner surface and, when the depending skirt is engaged in the cylindrical cavity, the peristaltic tube is engaged between the rollers and the inset lining such that rotation of the rotor pumps fluid through the peristaltic tube.

The thermal sensor assembly may include: a thermal sensor; a disposable metal tube connectable to the end of a passageway of the catheter to receive the fluid flowing therethrough; a metal thermal coupling member formed with a recess on one face for receiving the thermal sensor therein, a recess on the opposite face complementary to the shape of the metal tube for receiving the metal tube therein, and a relatively thin web between the two recesses; and a cover pressing the metal tube to the thermal coupling member.

The apparatus used in the technique for selectively thermally treating a targeted tissue may further include an air trapping element for trapping air circulating in the system, the air trapping element comprising a hung container controllably connectable to the closed system, the air trapping element being disposable.

A further technique for selectively thermally treating a targeted tissue adjacent to a subject's body cavity comprises the steps of: (a) inserting into a subject's body cavity a catheter, including a thermal treating section for thermally treating the targeted tissue adjacent the body cavity by circulating fluid of a predetermined temperature through the thermal treating section, the catheter being disposable; (b) alternately heating and cooling fluid circulating in a closed system including the catheter, and further including: a pump for pumping fluid through the closed system; thermal sensor assemblies for sensing the temperature of fluid circulating into and out of the catheter; and a temperature-setting device for alternately heating and cooling the fluid circulating therethrough, the temperature-setting device comprising: a tubular element having relatively low electrical conductivity and good thermal conductivity for receiving a quantity of circulating fluid, the tubular element having two ends connectable to electrically and thermally insulating tubular elements, the tubular element and the insulating tubular elements being disposable; a heating housing element for receiving the tubular element, the housing element detachably connectable to a heating source; and a cooling housing element for receiving the tubular element, the housing element detachably connectable to a cooling source, the tubular element transferable between the heating housing element and the cooling housing element, thereby alternately heating and cooling the fluid circulating therethrough.

The temperature-setting device may comprise: ( a ) a tubular element having relatively low electrical conductivity and good thermal conductivity for receiving a quantity of circulating fluid, the tubular element having two ends connectable to electrically and thermally insulating tubular elements, the tubular element and the insulating tubular elements being disposable; (b) a heating housing element for receiving the tubular element, the housing element including fastening elements, including: (i) a connector member having relatively good electrical conductivity, the connector member connectable to an electrical circuit; (ii) a fastening member for anchoring the tubular element between the fastening member and the connector member; (c) a transformer element connectable to the electrical circuit, the transformer element electrically connected to the controller; and (d) a cooling housing element comprising a thermal conducting member disposed within a bath of a cooling substance, the thermal conducting member having a recess for receiving the tubular element, the recess defining a relatively lowered area for trapping cold air, the thermal conducting member featuring extensions for increasing the contact area between the thermal conducting member and the cooling substance; the tubular element transferable between the heating housing element and the cooling housing element, thereby alternately heating and cooling the fluid circulating therethrough.

A technique for selectively thermally treating a targeted tissue adjacent a subject's body cavity may comprise the steps of: (a) inserting into a subject's body cavity a catheter, including a thermal treating section for thermally treating the targeted tissue adjacent the body cavity by circulating fluid of a predetermined temperature through the thermal treating section, the catheter being disposable; (b) alternately heating and cooling fluid circulating in a closed system including the catheter, and further including: a pump for pumping fluid through the closed system; thermal sensor assemblies for sensing the temperature of fluid circulating into and out of the catheter; and a temperature-setting device for alternately heating and cooling the fluid circulating therethrough, the temperature-setting device comprising: (i) a tubular element having relatively good thermal conductivity for receiving a quantity of circulating fluid, the tubular element having two ends connectable to thermally insulating tubular elements, the tubular element and the insulating tubular elements being disposable; (ii) a housing element for alternately heating and cooling the tubular element, the housing element comprising two thermal conducting plates, each having a recess, the recesses defining a tunnel for receiving the tubular element under pressure; (iii) a heating source detachably connectable to the housing element; and (iv) a cooling source detachably connectable to the housing element; the housing element alternately heating and cooling the tubular element, thereby alternately heating and cooling the fluid circulating therethrough.

The temperature-setting device may comprise: (a) a tubular element having relatively good thermal conductivity for receiving a quantity of circulating fluid, the tubular element having two ends connectable to thermally insulating tubular elements, the tubular element and the insulating tubular elements being disposable; (b) a housing element for alternately heating and cooling said tubular element, the housing element comprising two thermal conducting plates, each having a recess, the recesses defining a tunnel for receiving the tubular element under pressure; and (c) peltier heat pump elements detachably connectable to the thermal conducting plates, the peltier heat pump elements alternately heating and cooling the thermal conducting plates, thereby alternately heating and cooling the fluid circulating through the tubular element.

A technique for selectively thermally treating a targeted tissue adjacent a subject's body cavity may comprise the steps of: (a) inserting into a subject's body cavity a catheter, including a thermal treating section for thermally treating the targeted tissue adjacent the body cavity by circulating fluid of a predetermined temperature through the thermal treating section, the catheter being disposable; (b) heating fluid circulating in a closed system including the catheter, and further including: a pump for pumping fluid through the closed system; thermal sensor assemblies for sensing the temperature of fluid circulating into and out of the catheter; and a temperature-setting device for heating the fluid circulating therethrough, the temperature-setting device comprising: (i) a tubular element having relatively good thermal conductivity for receiving a quantity of circulating fluid, the tubular element having two ends connectable to thermally insulating tubular elements, the tubular element and the insulating tubular elements being disposable; (ii) a housing element for heating the tubular element, the housing element comprising two thermal conducting plates, each having a recess, the recesses defining a tunnel for receiving the tubular element under pressure; and (iii) a heating source detachably connectable to the housing element.

The catheter may include a proximal end formed with an inflatable thermal treating section to be located within the body cavity, and a distal end to be located externally of the body cavity; the catheter being formed with first and second passageways extending from the distal end to the inflatable thermal treating section for circulating fluid of a predetermined temperature through the inflatable thermal treating section.

The catheter may include a proximal end formed with an inflatable anchoring section for anchoring the catheter in the body cavity, distal end to be located externally of the body cavity, and an inflatable thermal treating section adjacent the proximal end to be located near the targeted tissue; the catheter being formed with first and second passageways extending from the distal end to the inflatable thermal treating section for circulating fluid of a predetermined temperature through the said inflatable thermal treating section, and a third passageway extending from the distal end to the inflatable anchoring section for introducing unheated fluid into the inflatable anchoring section.

The catheter may further include thermal insulation surrounding the first and second passageways from close to the distal end to close to the inflatable thermal section, the thermal insulation including a plurality of separate compartments containing a non-heated fluid, the compartments extending axially along the catheter, thereby the inflatable thermal treating section and the targeted tissue in its proximity may be heated to a desired temperature without correspondingly heating non-targeted tissues.

The temperature-setting device may comprise: an electrically conducting tubular element, a housing element for receiving the tubular element; and a transformer electrically connectable to the housing element. The tubular element functions as a resistor and heats the fluid circulating therethrough. Alternatively, the housing element comprises a thermal conducting member disposed within a bath of a cooling substance, the thermal conducting member having a recess for receiving the tubular element. The tubular element may alternately be placed in a housing element functioning as a heater and a housing element functioning as a cooler, thereby alternately heating or cooling the fluid circulating therethrough. The thermal treatment apparatus may comprise: the temperature-setting device ; a catheter insertable into a subject's body cavity, the catheter including a thermal treating section for thermally treating a targeted tissue; a pump; thermal sensor assemblies; and an air trapping element.

### BRIEF DESCRIPTION OF THE DRAWINGS

The invention is herein described, by way of example only, with reference to the accompanying drawings, wherein:
FIG. 1 is a schematic diagram illustrating an embodiment of a temperature-setting device and other main components of a thermal treatment apparatus according to the present invention;
FIG. 2a is a transverse sectional view of another embodiment of a temperature-setting device according to the present invention, along lines A--A in FIG. 2b.
FIG. 2b is a longitudinal sectional view of the embodiment shown in FIG. 2a, along line B--B.
FIG. 3a is a transverse sectional view of yet another embodiment of a temperature-setting device according to the present invention, along lines A--A in FIG. 3b.
FIG. 3b is a top view of the embodiment shown in FIG. 3a.
FIG. 4a is a side view, partially in longitudinal section, of a catheter according to the present invention;
FIG. 4b is a side view, partially in longitudinal section, of the catheter illustrated in FIG. 4a rotated 90°.
FIG. 5a, 5b and 5c are transverse sectional views of the catheter shown in FIG. 4b, along lines A--A, B--B and C--C.
FIG. 6 is a side view, partially in longitudinal section, of another embodiment of a catheter according to the present invention, in which the anchoring section is an integral part of the thermal treating section;
FIG. 7a, 7b and 7c are transverse sectional views of the catheter shown in FIG. 6, along lines A--A, B--B and C--C.
FIG. 8a is a longitudinal sectional view of a peristaltic pump according to the present invention, along line A--A in FIG. 8b;
FIG. 8b is a transverse sectional view of the peristaltic pump shown in FIG. 8a, along line B--B;
FIG. 8c is a fragmentary detail view along line C--C in FIG. 8b;
FIG. 9a is a top view of an embodiment of a thermal sensor assembly according to the present invention.
FIG. 9b is a fragmentary detail view of the thermal sensor assembly shown in FIG. 9a, along line B--B.
FIG. 9c is a transverse sectional view of the thermal sensor assembly shown in FIG. 9a, along line C--C.
FIG. 10a is a transverse sectional view of another embodiment of a thermal sensor assembly, along line A--A in FIG. 10b.
FIG. 10b is a longitudinal sectional view of the thermal sensor assembly shown in FIG. 10a, along line B--B.

### DESCRIPTION OF THE PREFERRED EMBODIMENTS

The present invention is of a thermal treatment apparatus for the treatment of the human body comprising a temperature-setting device for providing a desired temperature to a minimal quantity of fluid circulating in a closed system for selectively treating a targeted tissue by fluid-induced thermal therapy using a minimal quantity of circulating fluid and consuming minimal energy for heating (or cooling) the fluid.

The principles and operation of a thermal treatment apparatus including a temperature-setting device may be better understood with reference to the drawings and the accompanying description.

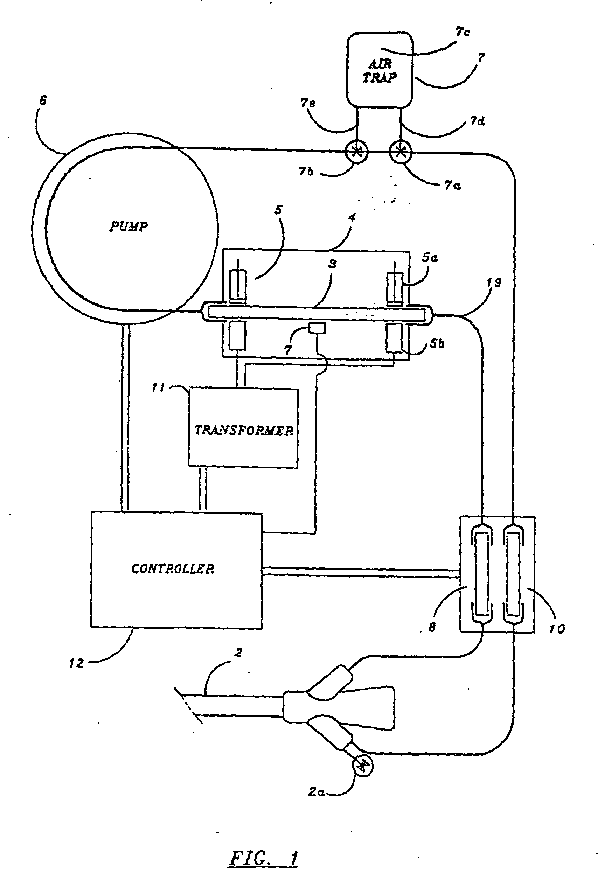

FIG. 1 illustrates a thermal treatment apparatus which uses fluid-induced thermal therapy for selectively treating a targeted tissue adjacent a subject's body cavity. The thermal treatment apparatus comprises a closed system, including: a temperature-setting device 4 for providing a desired temperature to a minimal quantity of fluid circulating through the system; a catheter 2 insertable into the subject's body cavity and connectable to temperature-setting device 4; a pump 6 for pumping the fluid through the system, the pump connectable to temperature-setting device 4; and preferably two thermal sensor assemblies 8 and 10 for measuring the temperature of fluid circulating into and out of catheter 2, respectively. Preferably, the closed system further includes an air trapping element 7 for trapping air circulating in the closed system.

The thermal treatment apparatus further includes a controller 12 for controlling the operation of temperature-setting device 4 and pump 6, preferably according to the temperature sensed by thermal sensor assemblies 8 and 10.

As shown in FIG. 1, a temperature-setting device according includes a resistive tubular element 3 having relatively low electrical conductivity and good thermal conductivity. Tubular element 3 may be made, for example, of stainless steel. Tubular element 3 is preferably disposable.

Preferably, temperature-setting device 4 further includes a housing element (not shown) having a recess for receiving tubular element 3. The housing element preferably includes two fastening elements 5 for mechanically anchoring tubular element 3 in the housing element, and electrically connecting the ends of tubular element 3 to a transformer 11. Each of fastening elements 5 comprises two components 5a and 5b. Component 5a preferably includes a fastening member such as a screwing element for anchoring tubular element 3 between component 5a and component 5b. Component 5b has relatively good electrical conductivity for allowing electrical connection between tubular element 3 and transformer 11.

Since tubular element 3 has relatively low electrical conductivity, it functions as a resistor and converts electrical energy to heat, thereby heating the fluid circulating therethrough. This configuration allows to circulate a minimal quantity of heated fluid through the system. Preferably, the quantity of circulating fluid is about 20-50 ml.

The voltage produced by transformer 11 is preferably very low, about 1-5 volts, in order to provide the necessary safety while using the device.

Preferably, temperature-setting device 4 further includes a temperature sensor 107 located at the housing element, for sensing the temperature of tubular element 3. The connection between sensor 107 and tubular element 3 is preferably established readily upon mounting of element 3 in the housing element, under pressure.

Sensor 107 is electrically connected to controller 12. Controller 12 may control the duty cycle of transformer 11 according to the temperature sensed by sensor 107, where the duty cycle refers to the time intervals during which transformer 11 provides electrical current or voltage to the system. Additionally, sensor 107 may function as a safety switch, so that when fluid circulation in the system is interrupted, sensor 107 heats up and consequently controller 12 preferably interrupts the operation of the overall system.

Alternatively, controller 12 may control the duty cycle of transformer 11 according to the temperature sensed by thermal sensor assemblies 8 and 10.

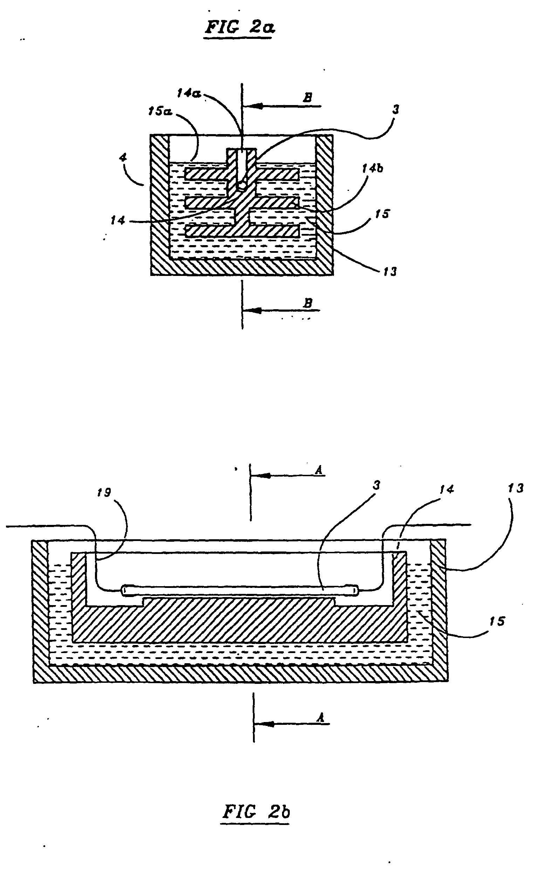

Referring now to FIGs. 2a and 2b, according to another embodiment of temperature-setting device 4, the housing element comprises a thermal conducting member 14 disposed within a bath 13 of a cooling substance 15, such as ice. Thermal conducting member 14 preferably features a recess 14a for receiving tubular element 3 therein. Recess 14a preferably defines a lowered area relative to the surface 15a of cooling substance 15, for trapping cold air. This configuration creates a local well of cold air defined by recess 14a, for efficiently cooling tubular element 3. Further, recess 14a may be used for receiving a quantity of fluid for improving the thermal coupling between thermal conducting member 14b and tubular element 3. Preferably, thermal conducting member 14 further features extensions 14b for increasing the area of contact between thermal conducting member 14 and cooling substance 15. Preferably, the housing element is stored under freezing conditions while not in use.

Temperature-setting device 4 may be used to alternately heat and cool a minimal quantity of fluid circulating through the system, by alternately placing tubular element 3 in embodiments of the device functioning as heaters (e.g. the configuration shown in FIG. 1) and embodiments functioning as coolers (e.g. the configuration shown in FIGs. 2a and 2b). While using the configuration of FIGs. 2a and 2b, transformer 11 is not used.

Referring now to FIGs. 3a and 3b, according to yet another embodiment of temperature-setting device 4, the housing element comprises two plates 16a and 16b, made of a material having relatively good thermal conductivity such as aluminum. Plates 16a and 16b are controllably connectable to heating and/or cooling sources 17 and 18, respectively. Heating and/or cooling sources 17 and 18 may be, for example, peltier heat pump elements. As shown, each of plates 16a and 16b may feature a recess, the recesses defining a tunnel for receiving tubular element 3 therein, preferably under pressure. This configuration allows better thermal coupling between thermal conducting plates 16a and 16b and tubular element 3.

While using this configuration, tubular element 3 is placed within the recess of plate 16b. Plate 16a is then placed on top of plate 16b, covering tubular element 3 so that tubular element 3 is preferably pressed within the tunnel defined by the recesses of plates 16a and 16b.

This configuration may be used as a heater and/or cooler, according to the function (heating or cooling) performed by sources 17 and 18. While using this configuration, transformer 11 is not used.

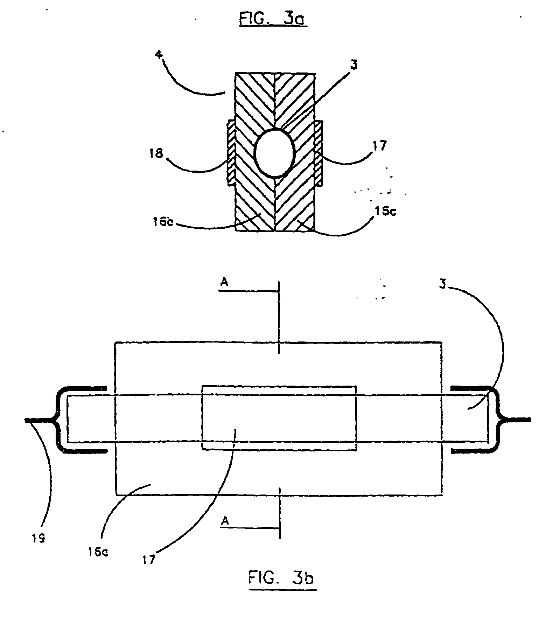

Referring now to FIGs. 4-7, thermal treatment apparatus according to the present invention further includes a catheter 2 connectable to temperature-setting device 4 and insertable into a subject's body cavity such as urinary bladder, prostatic urethra, uterus and esophagus, for selectively treating a targeted tissue within or near such cavity.

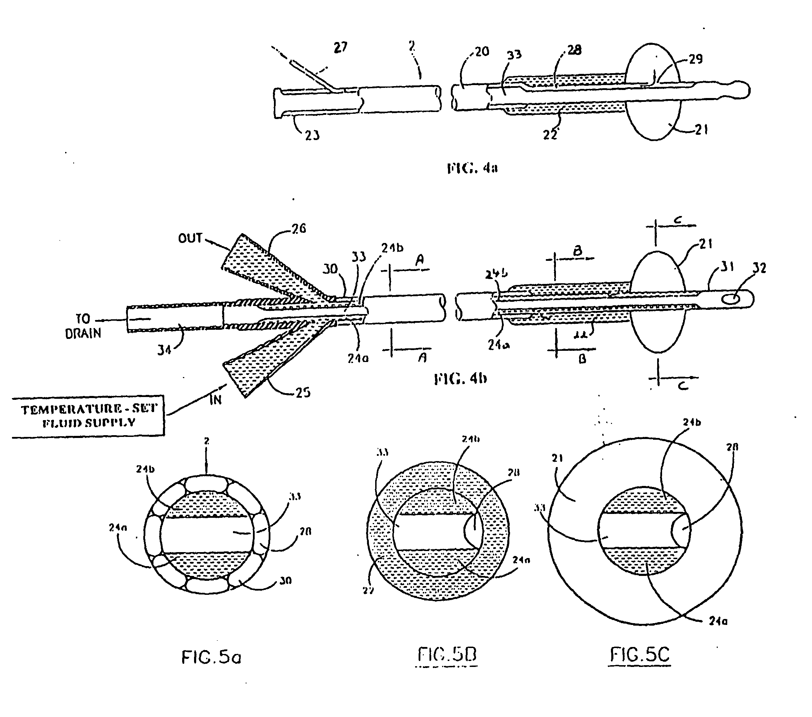

For example, selective treatment of a subject's prostate gland may be accomplished by inserting catheter 2 into the subject's urinary tract and anchoring catheter 2 within the subject's bladder. As shown in FIGs. 4a, 4b and 5a, 5b and 5c, catheter 2 preferably includes a long slender tube 20 formed with an inflatable anchoring section 21 at the proximal end for anchoring the catheter in the subject's body cavity, and thereby locating an inflatable cylindrical thermal treating section 22 near the targeted tissue to be treated. The length of thermal treating section 22 may preferably be adapted according to the distance between the bladder neck and the verumontanum of the particular patient. The distal end of catheter 23 is to be located externally of the subject's body so as to be readily accessible for inflating anchoring section 21 and thermal treating section 22.

A limited quantity of a fluid having a predetermined temperature is circulated through inflatable thermal treating section 22 via two passageways 24a and 24b having an inlet 25 and an outlet 26 at distal end 23 of the catheter. As shown in FIG. 1, a valve member 2a is connected to outlet 26 of the catheter for introducing fluid into the closed system.

Inflatable anchoring section 21 of the catheter is preferably inflated by an amount of unheated fluid introduced via inlet 27 at distal end 23 of the catheter. Inlet 27 preferably communicates with anchoring section 21 via an unheated-fluid passageway 28 and an opening 29.

The portion of the catheter from distal end 23 to inflatable thermal treating section 22 is preferably thermally insulated from the subject's untargeted tissues by means of insulating chambers 30 enclosing passageways 24a and 24b. One of insulating chambers 30 preferably communicates with unheated-fluid passageway 28 through which unheated air is applied to inflate anchoring section 21.

The catheter also includes an extension 31 at the proximal end, which extension is received within the subject's body cavity. Extension 31 is formed with an opening 32 for draining body fluids via draining passageway 33 passing through the length of the catheter and terminating in an outlet 34 attachable to a drain. Alternatively, passageway 33 may be used for introducing a drug into the subject's body cavity. Further, passageway 33 may be used for introducing a stiffening element for facilitating the insertion of the catheter to the subject's body cavity.

Catheter 2 and valve member 2a are preferably disposable.

According to another configuration (not shown), catheter 2 includes only one chamber 30 for communicating with inlet 27 and passageway 28 through which unheated air is applied to inflate anchoring section 21, so that passageways 24a and 24b along slender tube 20 are not insulated.

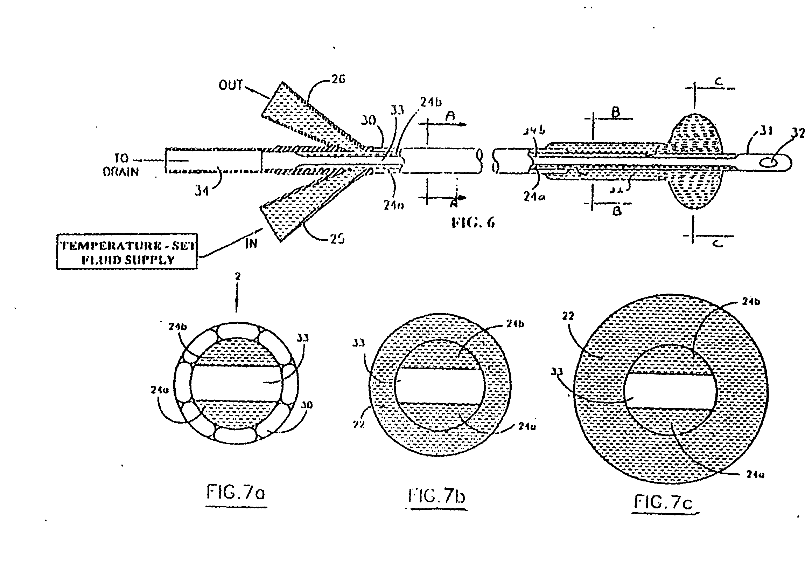

Yet another configuration of catheter 2 is shown in FIGs. 6 and 7a, 7b and 7c. In this configuration, anchoring section 21 is made as an integral part of thermal treating section 22, and passageway 28 is missing. Further, thermal treating section 22 may feature any geometrical shape, according to the specific geometry of the subject's body cavity. Such configuration may be used, for example, for endometrial ablation, where the geometry of thermal treating section 22 is adapted to fit the specific geometry of a subject's uterus, and insulating chambers 30 enable the use of a heating source for heating the fluid, which is completely external to the subject's body.

According to an additional configuration (not shown), anchoring section 21 is made as an integral part of thermal treating section 22, and all of chambers 30, inlet 27 and passageway 28 are missing, so that passageways 24a and 24b along slender tube 20 are not insulated. Inflatable thermal treating section 22 may feature any geometrical shape, according to the specific geometry of the subject's body cavity. Such configuration may be used, for example, for selectively heating a portion of a tissue treated by cryosurgery, thereby preserving that portion.

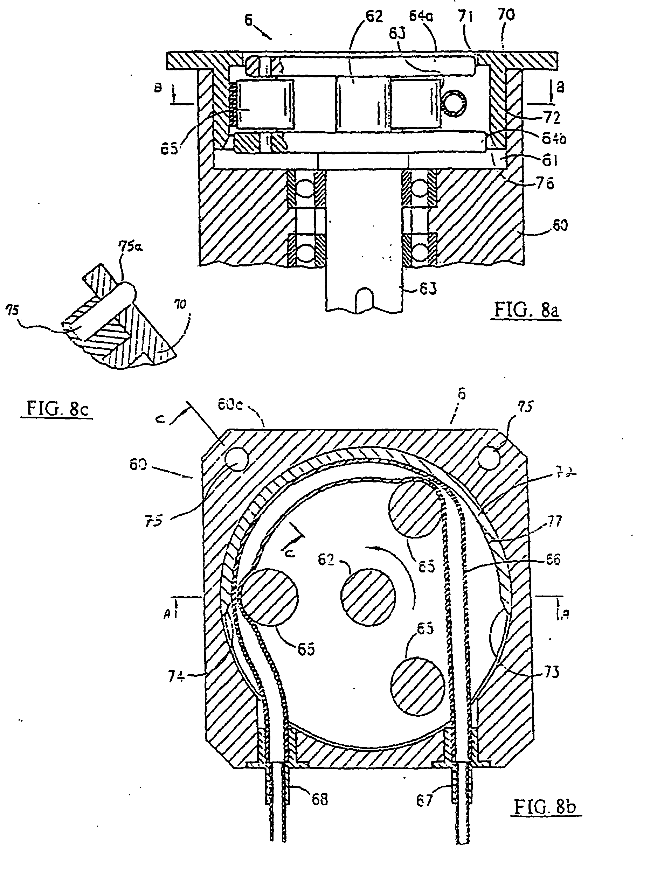

Referring now to FIGs. 8a, 8b and 8c, thermal treatment apparatus according to the present invention further includes a pump 6 connectable to catheter 2 and temperature-setting device 4. Pump 6 is preferably a peristaltic pump, and includes a housing 60 formed with a cylindrical cavity 61. Disposed within cylindrical cavity 61 is a rotor 62 connected by a drive shaft 63 to a gear motor (not shown) and including a pair of spaced discs 64a, 64b rotatably mounting between them a plurality (3) of rollers 65 within cavity 61. Also located within cylindrical cavity 61 is a peristaltic tube 66 which is engageable by roller 65 for pumping the fluid through the tube during the rotation of rotor 62. Assuming rotor 62 is rotated counter-clockwise in FIG. 8b, the fluid will be pumped through peristaltic tube 66 from an inlet nipple 67 to an outlet nipple 68.

As shown in FIG. 8a, housing 60 further includes a lid 70 with a large central opening 71 for accommodating disc 64a of rotor 62. Lid 70 is formed with a depending skirt 72 which extends into cylindrical cavity 61 such that peristaltic tube 66 is located between the inner surface of skirt 72 and rollers 65. Skirt 72 extends only for a part of the circumference of the lid, e.g., from 160° to 200°, to accommodate the inlet and outlet ends of peristaltic tube 66. As shown in FIG. 8b, skirt 72 extends slightly more than 180°; also, its leading edge 73 and its trailing edge 74 are tapered to provide a gradual application of pressure to the peristaltic tube by roller 65, and a gradual release of the pressure. Peristaltic tube 66 is preferably disposable.

Preferably, housing 60 features two or more pins 75, and lid 70 features two or more depressions 75a respectively for receiving pins 75 therein. Such configuration prevents the rotation of lid 70 during rotation of rotor 62.

The illustrated construction, including depending skirt 72, facilitates the assembly of the peristaltic pump with peristaltic tube 66 between skirt 72 and rollers 65. Thus, with the lid removed peristaltic tube 66 may be conveniently applied around rollers 65. Lid 70 may then be applied with its skirt 72 received between peristaltic tube 66 and the inner surface of cylindrical cavity 61 formed in housing 60, so as to squeeze the tube between skirt 72 and rollers 65. Accordingly, the lower edge of skirt 72 is tapered, as shown at 76 in FIG. 8a, for facilitating the application of the skirt.

The foregoing construction not only facilitates the assembly of the peristaltic pump, but also covers rollers 65 to minimize exposure to a person's fingers or the like. In addition, the thickness of skirt 72 influences the outlet pressure produced by the pump, so that lids 70 with different thickness of skirts 72 may be used to provide different outlet pressures. In addition, the inner surface of skirt 72 may be provided with one or more grooves, as shown at 77 in FIG. 8b, to produce a pulsatile output.

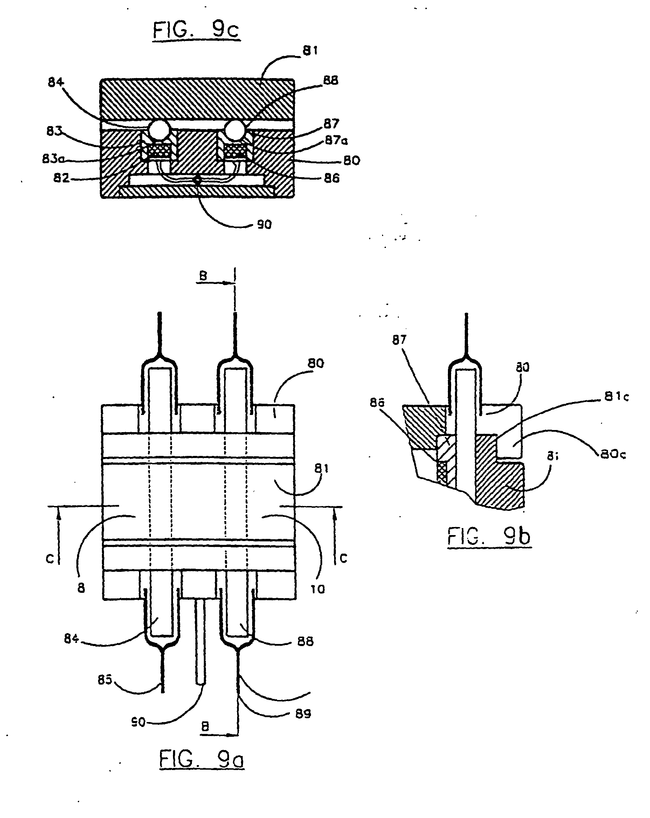

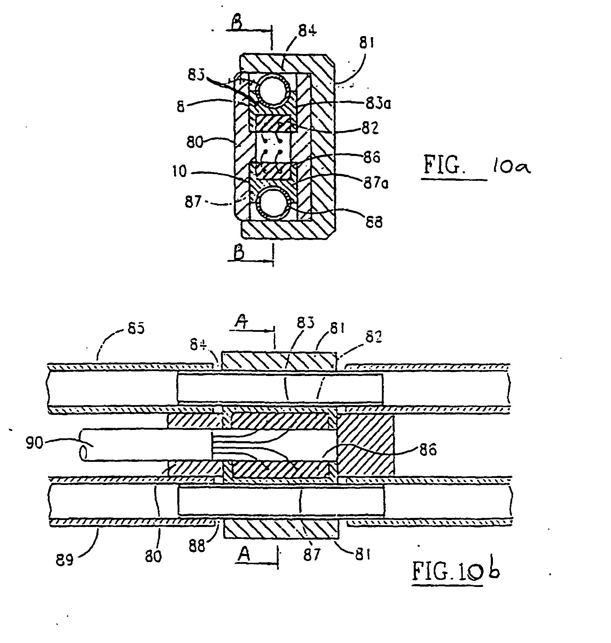

Referring now to FIGs. 9a, 9b, 9c, and 10a, 10b, thermal treatment apparatus according to the present invention preferably includes two thermal sensor assemblies 8 and 10 for measuring the temperature of fluid circulating into and out of catheter 2, respectively. Thermal sensor assemblies 8 and 10 are preferably enclosed within a common housing 80 and closed by a common cover 81. Thermal sensor assembly 8 near the inlet end of catheter 2 includes a thermal sensor element 82 received within a rectangular recess formed in a metal thermal coupling member 83. The opposite face of the coupling member is formed with a recess for receiving a metal tube 84 connectable to an inlet tube 85 near the inlet end of the catheter. Thermal sensor assembly 10 similarly includes a thermal sensor element 86 received within a recess formed in another metal thermal coupling member 87. The opposite face of member 87 is similarly formed with a recess for receiving a metal tube 88 adapted to be coupled to an outlet tube 89 near the outlet end of the catheter. Electrical connections are made to the two thermal sensor elements 82 and 86 via a cable 90 leading to controller 12 in FIG. 1. Metal tubes 84 and 88 and inlet and outlet tubes 85 and 89 are preferably disposable.

The two thermal coupling members 83, 87, as well as the two tubes 84, 88, are of a metal, such as stainless steel, having relatively good thermal conductivity. Coupling members 83, 87 include relatively thin web portions 83a, 87a, respectively, between thermal sensor elements 82, 86 and metal tubes 84, 88, so as to provide a good thermal coupling between the fluid flowing through the two metal tubes and their respective thermal sensor elements. Cover 81, fixed to the common housing 80 in any suitable manner, preferably presses metal tubes 84, 88 firmly against their respective metal coupling members 83, 87.

As shown in FIG. 9b, cover 81 may include extensions 81a, and housing 80 may respectively include depressions 80a for receiving extensions 81a of cover 81. Cover 81 is threaded into the chamber defined by depressions 80a of housing 80, pressing each of metal tubes 84 and 88 firmly against their respective metal coupling members 83 and 87.

Another possible configuration is shown in FIGs. 10a and 10b, wherein housing 80 features the shape of "H", and cover 81 presses each of metal tubes 84 and 88 firmly against their respective metal coupling members 83 and 87, and the central segment of housing element 80. Cover 81 may be fixed to the common H-shaped housing 80 in any suitable manner.

As shown in FIG. 1, thermal treatment apparatus according to the present invention may further include an air trapping element for trapping air circulating in the closed system. Air trapping element 7 may be in the form of a hung container 7c, which is preferably flexible and made of plastic, and controllably connectable to the closed system by means of two tubular elements 7d and 7e, and two valve members 7a and 7b. Container 7c, tubular elements 7d and 7e, and valve members 7a and 7b are preferably disposable.

Valve members 7a and 7b may controllably allow or block fluid circulation into and out of container 7c. When fluid is allowed to circulate through container 7c, the air found in the circulating fluid is trapped within the container.

Air trapping element 7 is preferably used for priming the system.

Thermal treatment apparatus according to the present invention preferably includes a disposable set, including: (a) different configurations of catheter 2 for different types of treatments; and (b) a tubing assembly, including: tubular element 3 of temperature-setting device 4; peristaltic tube 66 of pump 6; metal tubes 84 and 88 of thermal sensor assemblies 8 and 10; air trapping element 7; valve members such as 2a, 7a and 7b; and other tubular elements constituting the closed system of circulating fluid.

The overall operation of a thermal treatment apparatus including a temperature-setting device according to the present invention is as follows:

The configuration of catheter 2 is selected according to the specific type of treatment; catheter 2 is then connected to the disposable tubing assembly; tubular element 3 is placed in a suitable embodiment of temperature-setting device 4; peristaltic tube 66 is placed in pump 6; and metal tubes 84 and 88 are placed within thermal sensor assemblies 8 and 10 respectively.

Valve members 7a and 7b are set to allow circulation of fluid through air trapping element 7, and a minimal quantity (about 20-50 ml) of fluid is then introduced into the closed system via valve member 2a. Priming of the system is then carried out by activating pump 6 and circulating the fluid through air trapping element 7, thereby trapping air found in the system.

Valve members 7a and 7b are then set to block circulation of fluid through air trapping element 7, thereby directing the fluid to circulate through the main tube of the system.

An additional quantity of fluid is added at this stage to inflate thermal treating section 22 to its operational volume, if required.

Pump 6 is then switched off, and a quantity of fluid is pumped out of the system via valve member 2a, in order to deflate thermal treating section 22 and to allow the insertion of catheter 2 into the subject's body cavity.

Referring now to FIGs. 4 and 5, catheter 2 is inserted into a subject's body cavity untie inflatable anchoring section 21 at the proximal end passes through the neck of the cavity. An unheated fluid, preferably air, is introduced via inlet 27 and passageway 28 into the interior of anchoring section 21 to inflate it. This anchors section 21 in the subject's body cavity, whereupon heating section 22 of the catheter extends through the targeted tissue to be treated.

The quantity of fluid that was pumped out of the system for deflating thermal treating section 22 is now pumped into the system via valve member 2a for inflating thermal treating section 22 while placed within the subject's body cavity.

Referring now to FIGs. 6 and 7, wherein anchoring section 21 is made as an integral part of thermal treating section 22, and thermal treating section 22 features any geometrical shape. While using this configuration, the quantity of fluid that was pumped out of the system for deflating thermal treating section 22 is now pumped into the system via valve member 2a for inflating thermal treating section 22 while placed within the subject's body cavity. If required, an additional quantity of fluid is pumped into the system via valve member 2a so as to fill the particular body cavity, thereby efficiently heating or cooling the tissues therein.

The fluid is then circulated by peristaltic pump 6 through the closed system including thermal treating section 22 of the catheter and tubular element 3 of the temperature-setting device.

During circulation, tubular element 3 which is electrically connected to transformer 11 (FIG. 1), converts electrical energy to heat, thereby heating the fluid circulating therethrough. Alternatively, tubular element 3 may be placed in the embodiment shown in FIGs. 2a and 2b, thereby cooling the fluid circulating therethrough. Thus, temperature-setting device 4 may be used to alternately heat and cool the fluid circulating in the system by alternately placing tubular element 3 within embodiments of the device functioning as heaters and coolers.

Alternatively, tubular element 3 may be placed in the embodiment shown in FIGs. 3a and 3b, thereby alternately heating and cooling the circulating fluid using the same embodiment.

Sensor assemblies 8 and 10, electrically connected to controller 12, sense the temperature of fluid near the inlet and outlet ends of the catheter, respectively. Controller 12 maintain the desired temperature by controlling the duty cycle of transformer 11, and therefore the heating level of tubular element 3.

Sensor 7, electrically connected to controller 12, senses the temperature of tubular element 3. Controller 12 interrupts the operation of the overall system upon over--heating of tubular element 3.

While using the configurations of catheter 2 shown in FIGs. 4- 7, only inflated thermal treating section 22 of the catheter is effective to heat a targeted tissue, because of the thermal insulation provided by the unheated air within insulating chambers 30. While using the configuration shown in FIGs. 4 and 5, the insulation is further provided by anchoring section 21.

Accordingly, the fluid applied to inflatable thermal treating section 22 may be heated to a relatively high temperature for application to the targeted tissues, with less danger of unduly heating untargeted tissues contacted by the catheter. The inflation of thermal treating section 22 also presses that section firmly against the targeted tissue to be thermally treated thereby further enhancing the heating effects.

Drain opening 32 at the proximal end of the catheter, and passageway 33 through the catheter, provide a drain for body fluids or enable the introduction of drugs into the body cavity.

While the invention has been described with respect to a limited number of embodiments, it will be appreciated that many variations, modifications and other applications of the invention may be made within the scope of the appended claims.

## Claims

1. A thermal treatment apparatus for the treatment of the human body comprising a temperature-setting device (4) for providing a predetermined temperature to a quantity of fluid circulating in a closed-loop system of the thermal treatment apparatus, configured to heat and/or cool the circulating fluid to a predetermined temperature, **characterized by**:
a tubular thermal element (3) having a body that encloses a fluid flow passage and an axial length with opposing first and second ends, said tubular thermal element (3) having thermal conductivity and being sized and configured to allow circulating fluid to flow therethrough, wherein, in operation, said tubular thermal element body is configured to be heated and/or cooled and to transfer its thermal energy to heat and/or cool the circulating fluid to a predetermined temperature as the circulating fluid flows therethrough.

2. A thermal treatment apparatus according to Claim 1, wherein said tubular thermal element (3) ends are connectable to electrically and thermally insulating tubular elements, said tubular thermal element (3) and said insulating tubular elements being disposable after a single-use.

3. A thermal treatment apparatus according to Claim 2, said device further comprising.
an electrical circuit comprising a transformer element (11);
a housing element for receiving said thermal tubular element (3);
a connector member (5b) having relatively good electrical conductivity, said connector member (5b) connectable to the electrical circuit, and
a fastening member (5a) for anchoring said thermal tubular element (3) between said fastening member (5a) and said connector member (5b)

4. A thermal treatment apparatus according to Claim 3, further comprising a sensor (107) for sensing the temperature of said thermal tubular element (3).

5. A thermal treatment apparatus according to Claim 4, further comprising a controller (12), wherein said transformer element (11) and said sensor (107) are electrically connected to said controller (12); and wherein said controller (12) controls the duty cycle of said transformer element (11) responsive to the temperature sensed by said sensor (107).

6. A thermal treatment apparatus according to Claim 4, wherein said tubular thermal element (3) is a tubular heating element, said device further comprising a controller (12), wherein said transformer element (11) and said sensor (107) are electrically connected to said controller (12), and wherein said controller (12) is configured to interrupt the operation of said transformer element (11) and said electrical circuit to reduce the temperature of the tubular heating element (3) upon a certain value of temperature sensed by said sensor (107).

7. A thermal treatment apparatus according to any one of Claims 3-6, wherein said electrical circuit operates on about 1-5 volts.

8. A thermal treatment apparatus according to any one of Claims 4-6, wherein said housing element comprises a mounting region for receiving the thermal tubular element (3) and a sufficient electrical contact between said thermal tubular element (3) and said sensor (107) is established upon mounting of said tubular element in said mounting region.

9. A thermal treatment apparatus according to Claim 3, further comprising a a thermal conducting member (14) and a bath (13) of a cooling substance (15) configured to receive said tubular thermal element (3), said thermal conducting member (14) having a recess (14a) for receiving said thermal tubular element (3), said recess (14a) defining a relatively lowered region for trapping cold air, said thermal conducting member including extensions (14b) for increasing the contact area between said thermal conducting member (14) and said cooling substance (15), wherein said temperature-setting device (4) is configured to cool a quantity of liquid circulating through the thermal tubular element (3).

10. A thermal treatment apparatus according to Claim 3, wherein
said housing element is detachably connectable to a heating source (11); and
the device further comprises a cooling housing element for receiving said thermal tubular element detachable connectable to a cooling source (14), (15) or (18) said thermal tubular element (3) transferable between said heating housing element (4) or (17) and said cooling housing element (13) or (18), thereby alternately selectably heating or cooling the fluid circulating therethrough.

11. A thermal treatment apparatus according to any one of Claims 1 to 10, configured to circulate heated fluid in a circulating flow path, further comprising:
(a) a catheter (2) adapted for insertion into the human body, said catheter including a thermal treatment section for thermally treating targeted tissue in the human body by exposing targeted tissue to circulating fluid heated to a predetermined temperature that is contained in said catheter thermal treatment section;
(b) the temperature-setting device (4) being in fluid communication with said catheter (2) such that the circulating fluid flows through said tubular heating element (3) as it travels in the circulating flow path;
(c) a pump (6) operably associated with said tubular heating element (3) and catheter, said pump (6) configured to drive said fluid through said circulating flow path;
(d) a power source (11) operably associated with said tubular heating element (3); and
(e) a controller (12) operably associated with said power source (11),
wherein, in operation, said tubular heating element (3) body is controllably heated to thereby heat the circulating fluid traveling therethrough to the predetermined temperature.

12. A thermal treatment apparatus according to Claim 11 , wherein the volume of fluid in the circulating flow path is in the range of between about 20-50 ml.

13. A thermal treatment apparatus according to Claim 11 or 12, wherein said catheter (2) comprises a first inlet circulating channel (24a) and a separate second outlet circulating channel (24b), each in fluid communication with said thermal treatment section (22), said apparatus further comprising a plurality of temperature sensors, including a first temperature sensor (82) operably associated with said first fluid circulating channel (24a) and a second temperature sensor (86) operably associated with said second fluid circulating channel (10).

14. A thermal treatment apparatus according to Claim 13, wherein said plurality of sensors comprises a third temperature sensor (107) operably associated with said tubular heating element (3).

15. A thermal treatment apparatus according to any one of Claims 11-14, wherein said power source is configured to supply between about 1-5 volts to cause the tubular heating element (3) to raise to or maintain the circulating fluid at a desired temperature.

16. A thermal treatment apparatus according to any one of Claims 11-15, wherein, in operation, said tubular element (3) heats in response to voltage applied thereto, and wherein said controller (12) is configured to interrupt the application of voltage to said tubular heating element (3) responsive to when the circulating fluid reaches a predetermined elevated temperature, thereby providing a safety override to the operation of said thermal treatment apparatus.

17. A thermal treatment apparatus according to any of Claims 11 to 16, wherein said power source comprises a transformer element (11) electrically connected to said tubular element, said transformer element (11) being configured to supply power to said tubular heating element (3) to heat the tubular element.

18. A thermal treatment apparatus according to Claim 17, wherein said temperature-setting device (4) further comprises at least one temperature sensor (82, 86) for sensing a temperature of the circulating fluid in the circulating flow path, wherein said controller (12) is configured to control the operation of said transformer element (11), said controller (12) being electrically connected to at least one fluid temperature sensor (82, 86), said tubular element temperature sensor (107), and to said transformer element (11) such that said controller (12) is able to control said transformer element (11) according to the temperature of said fluid

19. A thermal treatment apparatus according to Claim 18, wherein said controller (12) controls the duty cycle of said transformer element (11) according to the temperature sensed by at least one of said temperature sensors (107, 8, 10).

20. A thermal treatment apparatus according to Claim 19, wherein said controller (12) interrupts the operation of said transformer element (11) and said power source upon detection of a certain value of temperature sensed by at least one of said temperature sensors (107, 8, 10).

21. A thermal treatment apparatus according to any one of Claims 14-20, further comprising a housing configured to hold said tubular heating element (3), and said temperature sensor (107), said apparatus further comprising a heat source (11) or (17), held in said housing, wherein said tubular heating element (3) is detachably connectable to the heat source (11 or 17).

22. A thermal treatment apparatus according to Claim 21, wherein said housing includes a recess for receiving the tubular heating element (3) and contact between said tubular heating element (3) and said temperature sensor (107) is established upon mounting of said tubular heating element (3) in said recess under pressure.

23. A thermal treatment apparatus according to any one of Claims 11-22, wherein said tubular heating element (3) has low electrical conductivity.

24. A thermal treatment apparatus according to any one of Claims 11-22, wherein said tubular heating element (3) comprises a stainless steel body.

25. A thermal treatment apparatus according to any of Claims 11-24, further comprising an air trap (7) operably associated with said circulating system flow path, wherein said air trap (7) is configured to remove air from said circulating flow path during operation.

26. A thermal treatment apparatus according to Claim 25, wherein said air trapping element is a hung container (7c) connectable to said circulating flow path.

27. A thermal treatment apparatus according to Claim 25 or 26, wherein said air trapping element is disposable.

28. A thermal treatment apparatus according to any one of Claims 11-27, wherein said tubular heating element (3) and said catheter (2) are disposable.

29. A thermal treatment apparatus according to any of Claims 11-28, wherein said catheter (2) has an elongated substantially cylindrical body having an axially extending outer wall with an outer surface and opposing proximate and distal ends and a central opening extending therethrough, wherein, in position, said proximate end portion is configured to enter a distance into the bladder of a subject,
said catheter comprising:
a drainage port (32) located on the proximate end portion thereof;
an inflatable anchoring balloon (21) located adjacent said drainage port;
an inflatable thermal treatment portion (22), that, in operation is configured to transmit a thermal heat treatment to the subject, positioned intermediate said anchoring balloon (21) and said distal end; and
a plurality of insulating chambers (30) positioned in circumferential spaced apart relationship inward of said outer wall and extending from a distal portion of said catheter to a desired distance therefrom to provide thermal insulation;
wherein said catheter comprises a first circulating fluid inlet channel (24a) and a second circulating fluid outlet channel (24b), each in fluid communication with said inflatable thermal treatment portion (22), and a third drainage channel (33) extending from said drainage port to a distal portion of said catheter to provide a urinary discharge flow path therebetween, and
wherein said tubular heating element (3) is in fluid communication with said first circulating fluid channel (24a) and is positioned spaced apart from said catheter such that it does not enter the body cavity of the subject during use.

30. A thermal treatment apparatus according to any of Claims 11-29, further comprising a thermal sensor assembly (8) for sensing the temperature of the fluid circulating in the circulating flow path into the catheter (2), said thermal sensor assembly (8) having two opposing ends, one of said ends connectable to said catheter (2) and the other of said ends connectable to said tubular heating element (3).

31. A thermal treatment apparatus according to Claims 29 and 30, further comprising a second thermal sensor assembly (10) for sensing the temperature of fluid circulating out of said catheter (2), said second thermal sensor assembly (10) having two opposing ends, one of said ends connectable to said catheter and the other of said ends connectable to said pump, said second thermal assembly (10) operably associated with said controller (12).

32. A thermal treatment apparatus according to Claim 31, further comprising first (85) and second (89) conduits connected to a respective one of said catheter first and second circulating fluid channels (24a, 24b), and first and second tubular thermal sensor members (84,88) attached to respective ones of the first and second fluid channels (24a, 24b), wherein said catheter (22), said tubular thermal element (3), said first and second conduits (85,89), and said first and second tubular thermal sensor members (84,88) are disposable after a single-use.

33. A thermal treatment apparatus according to any one of Claims 29-32, wherein said third drainage channel (33) is configured to direct a drug treatment from said catheter distal end portion to discharge at said drainage port.

34. A thermal treatment apparatus according to any one of Claims 29-32, wherein said third drainage channel (33) is configured to receive a stiffener member therein.

35. A thermal treatment apparatus according to any one of Claims 29-34, wherein said urinary drainage port (32) is located on the proximate end of said catheter (2), and wherein said anchoring portion (21) is spaced apart from said urinary drainage port (32) and located intermediate said drainage port (32) and said inflatable thermal treatment portion (22) along the length of said catheter, and wherein each of said urinary drainage port, said anchoring portion, and said inflatable thermal treatment portion are configured to be in fluid isolation from the others.

36. A thermal treatment apparatus according to any one of Claims 29-35, wherein said catheter (2) is configured to be insertable into a urethra, wherein, in position, said anchoring balloon (21) is configured to inflate to a bulbous shape to anchor said catheter (2) in location such that said anchoring balloon resides in the bladder to contact a portion of the bladder neck and hold said catheter in position, and wherein said inflatable thermal treatment portion is an ablation portion that expands to circumferentially contact the prostate tissue to thermally ablate prostate tissue in the body cavity.

37. A thermal treatment apparatus according to any one of Claims 29-36, wherein said catheter (2) is configured to direct air into said anchoring balloon (21) for inflation thereof, to direct circulating heated fluid into said thermal treatment portion (22) for expansion thereof, and to allow urine to freely flow from said drainage port (32) during operation.

38. A thermal treatment apparatus according to any one of Claims 29-37, wherein said catheter (2) is configured to direct heated fluid into said anchoring balloon (21) and said inflatable thermal treatment portion (22) to thereby inflate both.

39. A thermal treatment apparatus according to any one of Claims 29-38, wherein said thermal treatment apparatus is configured to ablate tissue, wherein said catheter inflatable thermal treatment portion (22) is a thermal ablation portion, wherein said anchoring balloon (21) and said inflatable thermal ablation portion (22) are in fluid communication, and wherein said catheter is configured to circulate heated fluid through both said inflatable thermal ablation portion and said anchoring balloon.

40. A thermal treatment apparatus according to any one of Claims 11-39, further comprising a cooling housing element adapted to receive the tubular heating element (3) into a container (14) having a bath of a coolant (15), wherein said tubular heating element (3) is detachably positioned in the housing to be in communication with said coolant (15) and/or to alternately heat and cool the fluid traveling in the circulating flow path.

## Patentansprüche

1. Wärmebehandlungsvorrichtung zur Behandlung des menschlichen Körpers, die eine Temperatureinstellungseinheit (4) umfasst, um einer Menge an Flüssigkeit, die in einem System mit geschlossenem Kreislauf der Wärmebehandlungsvorrichtung zirkuliert, eine vorgegebene Temperatur zu verleihen, wobei die Vorrichtung dazu eingerichtet ist, die zirkulierende Flüssigkeit auf eine vorgegebene Temperatur zu heizen und/oder zu kühlen, **gekennzeichnet durch**:
ein rohrförmiges thermisches Element (3), das einen Körper aufweist, der einen Flüssigkeitsflusskanal und eine axiale Länge mit gegenüberliegenden ersten und zweiten Enden einschließt, wobei das rohrförmige thermische Element (3) eine thermische Leitfähigkeit aufweist und so bemessen und eingerichtet ist, daß es den Durchfluss zirkulierender Flüssigkeit erlaubt, wobei der Körper des rohrförmigen thermischen Elements dazu eingerichtet ist, im Betrieb geheizt und/oder gekühlt zu werden und seine thermische Energie beim Durchfluss der zirkulierenden Flüssigkeit zu übertragen, um die zirkulierende Flüssigkeit auf eine vorgegebene Temperatur zu erhitzen und/oder zu kühlen.

2. Wärmebehandlungsvorrichtung nach Anspruch 1, wobei die Enden des rohrförmigen thermischen Elementes (3) mit elektrisch und thermisch isolierenden rohrförmigen Elementen verbunden werden können, wobei das rohrförmige thermische Element (3) und die isolierenden rohrförmigen Elemente nach einem einmaligem Gebrauch wegwerfbar sind.

3. Wärmebehandlungsvorrichtung nach Anspruch 2, wobei die Vorrichtung weiterhin umfasst:
eine elektrische Schaltung, die ein Wandlerelement (11) aufweist;
ein Gehäuseelement zur Aufnahme des thermischen rohrförmigen Elements (3);
ein Anschlussteil (5b) mit relativ guter elektrischer Leitfähigkeit, wobei das Anschlussteil (5b) an die elektrische Schaltung anschließbar ist; und
ein Befestigungsteil zum Befestigen des thermischen rohrförmigen Elements (3) zwischen dem Befestigungsteil (5a) und dem Anschlussteil (5b).

4. Wärmebehandlungsvorrichtung nach Anspruch 3, die weiterhin einen Sensor (107) zum Erfassen der Temperatur des rohrförmigen thermischen Elements (3) umfasst.

5. Wärmebehandlungsvorrichtung nach Anspruch 4, die weiterhin eine Steuereinheit (12) umfasst, wobei das Wandlerelement (11) und der Sensor (107) elektrisch mit der Steuereinheit (12) verbunden sind; und wobei die Steuereinheit (12) den Arbeitszyklus des Wandlerelements (11) in Abhängigkeit von der von dem Sensor (107) erfassten Temperatur steuert.

6. Wärmebehandlungsvorrichtung nach Anspruch 4, wobei das rohrförmige thermische Element (3) ein rohrförmiges Heizelement ist, die Vorrichtung weiter eine Steuereinheit (12) enthält, wobei das Wandlerelement (11) und der Sensor (107) elektrisch mit der Steuereinheit (12) verbunden sind und wobei die Steuereinheit (12) dazu eingerichtet ist, den Betrieb des Wandlerelements (11) und der elektrischen Schaltung zu unterbrechen, um die Temperatur des rohrförmigen Heizelements (3) bei einem bestimmten vom Sensor (107) erfassten Temperaturwert zu verringern.

7. Wärmebehandlungsvorrichtung nach einem der Ansprüche 3-6, wobei die elektrische Schaltung mit etwa 1-5 Volt arbeitet.

8. Wärmebehandlungsvorrichtung nach einem der Ansprüche 4-6, wobei das Gehäuseelement einen Montagebereich zur Aufnahme des thermischen rohrförmigen Elements (3) umfasst, wobei ein ausreichender elektrischer Kontakt zwischen dem thermischen rohrförmigen Element (3) und dem Sensor (107) durch die Montage des rohrförmigen Elements in dem Montagebereich hergestellt wird.

9. Wärmebehandlungsvorrichtung nach Anspruch 3, die weiterhin ein Wärmeleitungsbauteil (14) und ein Bad (13) mit einer Kühlsubstanz (15) umfasst, das zur Aufnahme des rohrförmigen thermischen Elements (3) eingerichtet ist, wobei das Wärmeleitungsbauteil (14) eine Aussparung (14a) zur Aufnahme des thermischen rohrförmigen Elements (3) aufweist, wobei die Aussparung (14a) einen im Verhältnis abgesenkten Bereich zum Aufnehmen kalter Luft definiert, wobei das Wärmeleitungsbauteil Ansätze (14b) zum Vergrößern der Kontaktfläche zwischen dem Wärmeleitungsbauteil (14) und der Kühlsubstanz (15) aufweist, und die Temperatureinstelleinheit (4) dazu eingerichtet ist, eine Menge an Flüssigkeit, die durch das thermische rohrförmige Element zirkuliert, zu kühlen.

10. Wärmebehandlungsvorrichtung nach Anspruch 3, wobei das Gehäuseelement abnehmbar an einer Heizquelle (11) befestigbar ist; und die Vorrichtung weiterhin ein Kühlgehäuseelement zur Aufnahme des thermischen rohrförmigen Elements, das lösbar mit einer Kältequelle (14), (15) oder (18) verbindbar ist, umfasst, wobei das thermische rohrförmige Element (3) zwischen dem heizenden Gehäuseelement, (4) oder (17), und dem kühlenden Gehäuseelement, (13) oder (18), übertragbar ist, wodurch die durchfließende Flüssigkeit wählbar wechselweise erhitzt oder gekühlt werden kann.

11. Wärmebehandlungsvorrichtung nach einem der Ansprüche 1 bis 10, eingerichtet zum Zirkulieren erhitzter Flüssigkeit in einem umlaufenden Fluss, die weiterhin umfasst.
(a) einen Katheter (2), der für das Einführen in den menschlichen Körper geeignet ist, wobei dieser Katheter einen Wärmebehandlungsbereich zur thermischen Behandlung von Zielgewebe im menschlichen Körper durch Exponieren des Zielgewebes der auf eine vorgegebene Temperatur erhitzten zirkulierenden Flüssigkeit, die sich in dem Wärmebehandlungsbereich des Katheters befindet, umfasst;
(b) die Temperatureinstellvorrichtung (4), die in Strömungsverbindung mit dem Katheter (2) steht, derart, dass die zirkulierende Flüssigkeit durch das rohrförmige Heizelement (3) fließt, wenn sie auf dem umlaufenden Fließweg fließt;
(c) eine Pumpe (6) die mit dem rohrförmigen thermischen Element (3) und dem Katheter funktionell verbunden ist, wobei die Pumpe (6) dazu ausgelegt ist, die Flüssigkeit durch den umlaufenden Fließweg zu pumpen;
(d) eine Spannungsquelle (11), die funktionell mit dem rohrförmigen Heizelement (3) verbunden ist; und
(e) eine Steuereinheit (12), die funktionell mit der Spannungsquelle (11) verbunden ist,
wobei der Körper des rohrförmigen Heizelements (3) im Betrieb kontrolliert beheizt wird, um hierdurch die durchfließende zirkulierende Flüssigkeit auf die vorgegebene Temperatur zu heizen.

12. Wärmebehandlungsvorrichtung nach Anspruch 11, wobei das Flüssigkeitsvolumen in dem umlaufenden Fließweg im Bereich zwischen 20-50 ml liegt.

13. Wärmebehandlungsvorrichtung nach Anspruch 11 oder 12, wobei der Katheter (2) einen ersten Einlassfließkanal (24a) und einen getrennten zweiten Auslassfließkanal (24b) umfasst, die beide in Strömungsverbindung mit dem Wärmebehandlungsbereich (22) sind, wobei die Vorrichtung weiterhin mehrere Temperatursensoren umfasst, die einen ersten Temperatursensor (82), der funktionell mit dem ersten Fließkanal (24a), und einen zweiten Temperatursensor (86), der funktionell mit dem zweiten zirkulierenden Fließkanal (10) verbunden ist, umfasst.

14. Wärmebehandlungsvorrichtung nach Anspruch 13, wobei die mehreren Sensoren einen dritten Sensor (107) umfassen, der funktionell mit dem rohrförmigen Heizelement (3) verbunden ist.

15. Wärmebehandlungsvorrichtung nach einem der Ansprüche 11-14, wobei die Spannungsquelle dazu eingerichtet ist, zwischen etwa 1-5 Volt zu liefern, um zu bewirken, dass das rohrförmige Heizelement (3) die umlaufende Flüssigkeit auf eine gewünschte Temperatur zu bringt oder dort hält.

16. Wärmebehandlungsvorrichtung nach einem der Ansprüche 11-15, wobei das rohrförmige Element (3) im Betrieb in Abhängigkeit von daran angelegter Spannung heizt, und wobei die Steuereinheit (12) dazu ausgelegt ist, das Anlegen von Spannung an das rohrförmige Heizelement in Reaktion darauf zu unterbrechen, daß die umlaufende Flüssigkeit eine vorgegebene höhere Temperatur erreicht hat, wodurch eine Sicherheitsbeeinflussung des Betriebs der Warmebehandlungsvorrichtung bereitgestellt wird.

17. Wärmebehandlungsvorrichtung nach einem der Ansprüche 11 bis 16, wobei die Spannungsquelle ein Wandlerelement (11) umfasst, das elektrisch mit dem rohrförmigen Element verbunden ist, wobei das Wandlerelement (11) dazu eingerichtet ist, das rohrförmige Heizelement mit Spannung zum Beheizen des rohrförmigen Elements (3) zu versorgen.

18. Wärmebehandlungsvorrichtung nach Anspruch 17, wobei die Temperatureinstellungseinheit (4) weiterhin mindestens einen Temperatursensor (82, 86) zum Erfassen der Temperatur der zirkulierenden Flüssigkeit in dem umlaufenden Fließweg umfasst, wobei die Steuereinheit (12) dazu eingerichtet ist, den Betrieb des Wandlerelements (11) zu steuern, wobei die Steuereinheit (12) elektrisch mit mindestens mit einem Flüssigkeitssensor (82, 86), dem Temperatursensor des rohrförmigen Elements (107) und dem Wandlerelement (11) verbunden ist, derart, dass die Steuereinheit (12) in der Lage ist, das Wandlerelement (11) je nach der Temperatur der Flüssigkeit zu steuern.

19. Warmebehandlungsvorrichtung nach Anspruch 18, wobei die Steuereinheit (12) den Arbeitszyklus des Wandlerelements (11) steuert, je nach der Temperatur, die durch mindestens einen der Sensoren (107, 8, 10) erfasst wird.

20. Wärmebehandlungsvorrichtung nach Anspruch 19, wobei die Steuereinheit (12) den Betrieb des Wandlerelements (11) und die Spannungsquelle bei Messung eines bestimmten Temperaturwertes, der an mindestens einem der Temperatursensoren (107, 8, 10) gemessen wird, unterbricht

21. Wärmebehandlungsvorrichtung nach einem der Ansprüche 14-20, die weiterhin ein Gehäuse umfasst, das zur Befestigung des rohrförmigen Heizelements (13) und des Temperatursensors (107) eingerichtet ist, wobei die Vorrichtung weiterhin eine in dem Gehäuse befestigte Heizquelle (11) oder (17) umfasst, die in dem Gehäuse befestigt ist,
wobei das rohrförmige Heizelement (3) abnehmbar mit der Heizquelle (11 oder 17) verbunden werden kann

22. Wärmebehandlungsvorrichtung nach Anspruch 21, wobei das Gehäuse eine Aussparung zur Aufnahme des rohrförmigen Heizelements (3) und Herstellen eines Kontakts zwischen dem rohrförmigen Heizelement (3) und dem Temperatursensor (107) bei Montage des rohrförmigen Heizelements (3) in der Aussparung unter Druck umfasst.

23. Wärmebehandlungsvorrichtung nach einem der Ansprüche 11-22, wobei das rohrförmige Heizelement (3) eine geringe elektrische Leitfähigkeit aufweist.

24. Wärmebehandlungsvorrichtung nach einem der Ansprüche 11-22, wobei das rohrförmige Heizelement (3) einen Körper aus rostfreiem Stahl aufweist.

25. Wärmebehandlungsvorrichtung nach einem der Ansprüche 11-24, die weiterhin eine Luftfalle (7) umfasst, die funktionell mit dem umlaufenden Fließweg verbunden ist, wobei die Luftfalle (7) dazu eingerichtet ist, Luft aus dem umlaufenden Fließweg während dem Betrieb zu entfernen.

26. Wärmebehandlungsvorrichtung nach Anspruch 25, wobei die Luftfalle ein eingehängter Behälter (7c) ist, der mit dem umlaufenden Fließweg verbindbar ist.

27. Wärmebehandlungsvorrichtung nach Anspruch 25 öder 26, wobei die Luftfalle wegwerfbar ist.

28. Wärmebehandlungsvorrichtung nach einem der Ansprüche 11-27. wobei das rohrförmige Heizelement (3) und der Katheter (2) wegwerfbar sind.

29. Wärmebehandlungsvorrichtung nach einem der Ansprüche 11-28, wobei der Katheter (2) einen länglichen, grundsätzlich zylindrischen Körper aufweist, der eine sich axial erstreckende äußere Wand mit einer äußeren Oberfläche, ein proximales und ein distales Ende, die sich gegenüberliegen, sich hierdurch erstreckende zentrale Öffnung, aufweist, wobei der proximale Endbereich in Position dazu eingerichtet ist, eine Strecke in die Harnblase einer Person einzudringen,
wobei der Katheter folgendes umfasst:
einen Zuflussanschluss (32), der an dem proximalen Endbereich angeordnet ist;
einen aufblasbaren Verankerungsballon (21), der benachbart zum Zuflussanschluss angeordnet ist;
einen aufblasbaren Wärmebehandlungsbereich (22), der dazu eingerichtet ist, im Betrieb der Person eine Wärmebehandlung zu geben, wobei diese zwischen dem Verankerungsballon (21) und dem distalen Ende angeordnet ist, und
mehrere isolierende Kammern (30), die in umfangsmäßig beabstandeter Weise innerhalb der äußeren Wand angeordnet sind, und sich von einem distalen Bereich des Katheters zu einer gewünschten Entfernung von diesem erstrecken, um thermische Isolierung herzustellen;
wobei der Katheter einen ersten umlaufenden Einlassfließkanal (24a) und einen zweiten umlaufenden Auslassfließkanal (24b) umfasst, von denen jeder in Strömungsverbindung mit dem aufblasbaren Wärmebehandlungsbereich (22), und einen dritten Ausflusskanal (33), der sich vom Zuflussanschluss zu einem distalen Bereich des Katheters erstreckt, um dazwischen einen Harnabflusspfad herzustellen, und
wobei das Heizelement (3) in Strömungsverbindung mit dem ersten umlaufenden Fließkanal (24a) steht und beabstandet vom Katheter angeordnet ist, derart, dass es bei der Benutzung nicht in die Körperhöhle der Person eintritt.

30. Wärmebehandlungsvorrichtung nach einem der Ansprüche 11-29, die weiterhin eine thermischen Sensoranordnung (8) zum Erfassen der Temperatur der Flüssigkeit, die im umlaufenden Fließweg in den Katheter (2) fließt, umfasst, wobei die thermische Sensoranordnung (8) zwei gegenüberliegende Enden aufweist, wobei eines der Enden mit dem Katheter (2) und das andere der Enden mit dem rohrförmigen Heizelement (3) verbindbar ist.

31. Wärmebehandlungsvorrichtung nach Anspruch 29 und 30, die weiterhin eine zweite thermische Sensoranordnung (10) zum Erfassen der Temperatur der Flüssigkeit, die aus dem Katheter (2) fließt, umfasst, wobei die zweite thermische Sensoranordnung (10) zwei gegenüberliegende Enden aufweist, wobei eines der Enden mit dem Katheter und das andere der Enden mit der Pumpe verbindbar ist, wobei die zweite thermische Anordnung (10) funktionell mit der Steuereinheit (12) verbunden ist.

32. Wärmebehandlungsvorrichtung nach Anspruch 31, die weiterhin eine erste (85) und eine zweite (89) Leitung, die mit dem entsprechenden ersten bzw. zweiten umlaufenden Katheterflüssigkeitskanal (24a, 24b) verbunden sind, und einen ersten und einen zweiten rohrförmigen thermischen Sensorteil (84, 88) umfasst, die an dem entsprechenden ersten bzw. zweiten Flüssigkeitskanal (24a, 24b) befestigt sind, wobei der Katheter (22), das rohrförmige thermische Element (3), die erste und zweite Leitung (85, 89) und der erste und der zweite rohrförmige thermische Sensorteil (84, 88) nach Einmalgebrauch wegwerfbar sind.

33. Wärmebehandlungsvorrichtung nach einem der Ansprüche 29-32, wobei der dritte Ausflusskanal (33) dazu eingerichtet ist, eine medikamentöse Behandlung von dem distalen Endbereich des Katheters zum Austritt an dem Ausflusskanal zu führen.

34. Wärmebehandlungsvorrichtung nach einem der Ansprüche 29-32, wobei der dritte Ausflusskanal (33) zur Aufnahme eines Versteifungsteils eingerichtet ist.

35. Wärmebehandlungsvorrichtung nach einem der Ansprüche 29-34, wobei der Harnausflusskanal (32) am proximalen Ende des Katheters (2) angeordnet ist und wobei der Verankerungsbereich (21) von dem Harnabflussanschluss (32) beabstandet ist und zwischen dem Ausflussanschluss (32) und dem aufblasbaren Wärmebehandlungsbereich (22) entlang des Katheters angeordnet ist, und wobei der Harnausflussanschluss, der Verankerungsbereich und der aufblasbare Wärmebehandlungsbereich jeweils strömungsmäßig von den anderen isoliert eingerichtet ist.

36. Wärmebehandlungsvorrichtung nach einem der Ansprüche 29-35, wobei der Katheter (2) dazu eingerichtet ist, in eine Harnröhre eingeführt werden zu können, wobei der Verankerungsballon (21) so eingerichtet ist, dass er sich in Position in eine ballige Form zum Verankern des Katheters (2) aufbläst, derart, dass der Verankerungsballon in der Harnblase bleibt, um einen Teil der Harnblasenverjüngung zu beruhren und den Katheter (2) in Position zu halten, wobei der aufblasbare Wärmebehandlungsbereich ein Ablationsbereich ist, der sich ausdehnt um ringsum das Prostatagewebe zu berühren, um thermisch Prostatagewebe in der Körperhöhle abzutragen.

37. Wärmebehandlungsvorrichtung nach einem der Ansprüche 29-36, wobei der Katheter (2) dazu eingerichtet ist, bei Betrieb Luft in den Verankerungsballon (21) zum Aufblasen desselben zu führen, erwärmte umlaufende Flüssigkeit in den Wärmebehandlungsbereich (22) zum Expandieren desselben, und das freie Abfließen von Urin aus dem Ausflussanschluss (32) zu ermöglichen.

38. Wärmebehandlungsvorrichtung nach einem der Ansprüche 29-37, wobei der Katheter (2) dazu eingerichtet ist, erwärmte Flüssigkeit in den Verankerungsballon (21) und den aufblasbaren Wärmebehandlungsbereich (22) zu führen, um hierdurch beide aufzublasen.

39. Wärmebehandlungsvorrichtung nach einem der Ansprüche 29-38, wobei die Wärmebehandlungsvorrichtung dazu eingerichtet ist, Gewebe abzutragen, wobei der aufblasbare Katheterwärmebehandlungsbereich (22) ein thermischer Ablationsbereich ist, wobei der Verankerungsballon (21) und der aufblasbare Ablationsbereich (22) in Strömungsverbindung stehen, und wobei der Katheter dazu eingerichtet ist, erwärmte Flüssigkeit sowohl durch den aufblasbaren Wärmebehandlungsbereich als auch durch den Verankerungsballon zu zirkulieren.

40. Wärmebehandlungsvorrichtung nach einem der Ansprüche 11-39, die weiterhin ein Kühlgehäuseelement umfasst, das geeignet ist, um das rohrförmige Heizelement (3) in einen Behälter (14) mit einem Bad eines Kühlmittels (15) aufzunehmen, wobei das rohrförmige Heizelement (3) abnehmbar im Gehäuse angeordnet ist, um in Verbindung mit dem Kühlmittel (15) zu stehen und/oder die den umlaufenden Fließweg durchströmende Flüssigkeit wechselweise zu heizen und zu kühlen.

## Revendications

1. Appareil de traitement thermique pour le traitement du corps humain comprenant un dispositif de réglage de température (4) pour fournir une température prédéterminée à une quantité de fluide circulant dans un système en boucle fermée de l'appareil de traitement thermique, configuré pour chauffer et/ou refroidir le fluide en circulation à une température prédéterminée, **caractérisé par** :
un élément thermique tubulaire (3) ayant un corps qui renferme un passage de flux de fluide et une longueur axiale avec une première extrémité et une seconde extrémité qui sont opposées, ledit élément thermique tubulaire (3) ayant une conductivité thermique et étant dimensionné et configuré pour permettre au fluide en circulation de s'écouler à travers celui-ci, dans lequel, en pratique, ledit corps d'élément thermique tubulaire est configuré pour être chauffé et/ou refroidi et pour transférer son énergie thermique pour chauffer et/ou refroidir le fluide en circulation à une température prédéterminée au fur et à mesure que le fluide en circulation s'écoule à travers celui-ci.

2. Appareil de traitement thermique selon la revendication 1, dans lequel les extrémités dudit élément thermique tubulaire (3) peuvent être connectées pour isoler électriquement et thermiquement les éléments tubulaires, ledit élément thermique tubulaire (3) et lesdits éléments tubulaires d'isolation étant jetables après une seule utilisation.

3. Appareil de traitement thermique selon la revendication 2, ledit dispositif comprenant en outre :
un circuit électrique comprenant un élément de transformateur (11) ;
un élément de boîtier pour recevoir ledit élément tubulaire thermique (3) ;
un membre connecteur (5b) ayant une conductivité électrique relativement bonne, ledit membre connecteur (5b) pouvant être connecté au circuit électrique ; et
un membre de fixation (5a) pour ancrer ledit élément tubulaire thermique (3) entre ledit élément de fixation (5a) et ledit membre de connecteur (5b).

4. Appareil de traitement thermique selon la revendication 3, comprenant en outre un capteur (107) pour détecter la température dudit élément tubulaire thermique (3).

5. Appareil de traitement thermique selon la revendication 4, comprenant en outre un contrôleur (12), dans lequel ledit élément de transformateur (11) et ledit capteur (107) sont électriquement connectés au dit contrôleur (12) ; et dans lequel ledit contrôleur (12) contrôle le cycle d'usage dudit élément de transformateur (11) en réponse à la température détectée par ledit capteur (107).

6. Appareil de traitement thermique selon la revendication 4, dans lequel ledit élément thermique tubulaire (3) est un élément de chauffage tubulaire, ledit dispositif comprenant en outre un contrôleur (12), dans lequel ledit élément de transformateur (11) et ledit capteur (107) sont connectés électriquement au dit contrôleur (12), et dans lequel ledit contrôleur (12) est configuré pour interrompre le fonctionnement dudit élément de transformateur (11) et dudit circuit électrique pour réduire la température de l'élément de chauffage tubulaire (3) à une certaine valeur de température détectée par le capteur (107).

7. Appareil de traitement thermique selon l'une quelconque des revendications 3 à 6, dans lequel ledit circuit électrique fonctionne à environ 1 V à 5 V.

8. Appareil de traitement thermique selon l'une quelconque des revendications 4 à 6, dans lequel ledit élément de boîtier comprend une région de montage pour recevoir l'élément tubulaire thermique (3) et un contact électrique suffisant entre ledit élément tubulaire thermique (3) et ledit capteur (107) est établi au montage dudit élément tubulaire dans ladite région de montage.

9. Appareil de traitement thermique selon la revendication 3, comprenant en outre un membre conducteur thermique (14) et un bain (13) d'une substance de refroidissement (15) configuré pour recevoir ledit élément thermique tubulaire (3), ledit élément conducteur thermique (14) ayant un renfoncement (14a) pour recevoir ledit élément tubulaire thermique (3), ledit renfoncement (14a) définissant une région relativement abaissée pour piéger l'air froid, ledit membre conducteur thermique comprenant des extensions (14b) pour accroître la zone de contact entre ledit membre conducteur thermique (14) et ladite substance de refroidissement (15), dans lequel ledit dispositif de réglage de température (4) est configuré pour refroidir une quantité de liquide en circulation à travers l'élément tubulaire thermique (3).

10. Appareil de traitement thermique selon la revendication 3, dans lequel
ledit élément de boîtier est connectable de manière amovible à une source de chauffage (11) ; et
le dispositif comprend en outre un élément de boîtier de refroidissement pour recevoir ledit élément tubulaire thermique connectable de manière amovible à une source de refroidissement (14), (15) ou (18), ledit élément tubulaire thermique (3) transférable entre ledit élément de boîtier de chauffage (4) ou (17) et ledit élément de boîtier de refroidissement (13) ou (18), pour ainsi chauffer ou refroidir alternativement de manière sélectionnable le fluide en circulation à travers celui-ci.

11. Appareil de traitement thermique selon l'une quelconque des revendications 1 à 10, configuré pour faire circuler du fluide chauffé dans un trajet de flux circulant, comprenant en outre :
(a) un cathéter (2) adapté pour être inséré dans le corps humain, ledit cathéter comprenant une partie de traitement thermique pour traiter thermiquement des tissus ciblés dans le corps humain en exposant les tissus ciblés au fluide en circulation chauffé à une température prédéterminée qui est contenu dans ladite partie de traitement thermique du cathéter ;
(b) le dispositif de réglage de température (4) étant en communication fluide avec ledit cathéter (2) de sorte que le fluide en circulation s'écoule à travers ledit élément de chauffage tubulaire (3) au fur et à mesure qu'il parcourt le trajet de flux circulant ;
(c) une pompe (6) associée de manière opérationnelle au dit élément de chauffage tubulaire (3) et au cathéter, ladite pompe (6) étant configurée pour entraîner ledit fluide à travers ledit trajet de flux circulant ;
(d) une source d'alimentation électrique (11) associée de manière opérationnelle au dit élément de chauffage tubulaire (3) ; et
(e) un contrôleur (12) associé de manière opérationnelle à ladite source d'alimentation électrique (11),
dans lequel, en pratique, le corps dudit élément de chauffage tubulaire (3) est chauffé de manière contrôlable pour ainsi chauffer le fluide en circulation qui le traverse à la température prédéterminée.

12. Appareil de traitement thermique selon la revendication 11, dans lequel le volume de fluide dans le trajet de flux circulant est compris entre environ 20 ml et 50 ml.

13. Appareil de traitement thermique selon la revendication 11 ou 12, dans lequel ledit cathéter (2) comprend un premier canal de circulation d'entrée (24a) et un deuxième canal de circulation de sortie distinct (24b), chacun étant en communication fluide avec ladite partie de traitement thermique (22), ledit appareil comprenant en outre une pluralité de capteurs de température comprenant un premier capteur de température (82) associé de manière opérationnelle au dit premier canal de circulation de fluide (24a) et un deuxième capteur de température (86) associé de manière opérationnelle au dit deuxième canal de circulation de fluide (10).

14. Appareil de traitement thermique selon la revendication 13, dans lequel ladite pluralité de capteurs comprend un troisième capteur de température (107) associé de manière opérationnelle au dit élément de chauffage tubulaire (3).

15. Appareil de traitement thermique selon l'une quelconque des revendications 11 à 14, dans lequel ladite source d'alimentation électrique est configurée pour fournir entre environ 1 V et 5 V pour amener l'élément de chauffage tubulaire (3) à augmenter ou à maintenir le fluide en circulation à une température souhaitée.

16. Appareil de traitement thermique selon l'une quelconque des revendications 11 à 15, dans lequel, en pratique, ledit élément tubulaire (3) chauffe en réponse à la tension appliquée sur celui-ci, dans lequel ledit contrôleur (12) est configuré pour interrompre l'application de la tension sur ledit élément de chauffage tubulaire (3) en réponse au moment où le fluide de circulation atteint une température élevée prédéterminée, ce qui assure un arrêt par sécurité du fonctionnement dudit appareil de traitement thermique.

17. Appareil de traitement thermique selon l'une quelconque des revendications 11 à 16, dans lequel ladite source d'alimentation électrique comprend un élément de transformateur (11) connecté électriquement au dit élément tubulaire, ledit élément de transformateur (11) étant configuré pour assurer l'alimentation électrique dudit élément de chauffage tubulaire (3) pour chauffer l'élément tubulaire.

18. Appareil de traitement thermique selon la revendication 17, dans lequel ledit dispositif de réglage de température (4) comprend en outre au moins un capteur de température (82, 86) pour détecter une température du fluide en circulation dans le trajet de flux circulant, dans lequel ledit contrôleur (12) est configuré pour contrôler le fonctionnement dudit élément de transformateur (11), ledit contrôleur (12) étant connecté électriquement à au moins un capteur de température de fluide (82, 86), au dit capteur de température d'élément tubulaire (107) et au dit élément de transformateur (11) de sorte que ledit contrôleur (12) est capable de contrôler ledit élément de transformateur (11) en fonction de la température dudit fluide.

19. Appareil de traitement thermique selon la revendication 18, dans lequel ledit contrôleur (12) contrôle le cycle d'usage dudit élément de transformateur (11) en fonction de la température détectée par au moins un desdits capteurs de température (107, 8, 10).

20. Appareil de traitement thermique selon la revendication 19, dans lequel ledit contrôleur (12) interrompt le fonctionnement dudit élément de transformateur (11) et de ladite source d'alimentation électrique à la détection d'une certaine valeur de température détectée par au moins l'un desdits capteurs de température (107, 8, 10).

21. Appareil de traitement thermique selon l'une quelconque des revendications 14 à 20, comprenant en outre un boîtier configuré pour contenir ledit élément de chauffage tubulaire (3), et ledit capteur de température (107), ledit appareil comprenant en outre une source de chaleur (11) ou (17), contenue dans ledit boîtier, dans lequel ledit élément de chauffage tubulaire (3) est connectable de manière amovible à ladite source de chaleur (11 ou 17).

22. Appareil de traitement thermique selon la revendication 21, dans lequel ledit boîtier comprend un renfoncement pour recevoir l'élément de chauffage tubulaire (3) et un contact entre ledit élément de chauffage tubulaire (3) et ledit capteur de température (107) est établi au montage dudit élément de chauffage tubulaire (3) dans ledit renfoncement sous pression.

23. Appareil de traitement thermique selon l'une quelconque des revendications 11 à 22, dans lequel ledit élément de chauffage tubulaire (3) a une faible conductivité électrique.

24. Appareil de traitement thermique selon l'une quelconque des revendications 11 à 22, dans lequel ledit élément de chauffage tubulaire (3) comprend un corps en acier inoxydable.

25. Appareil de traitement thermique selon l'une quelconque des revendications 11 à 24 comprenant en outre un collecteur d'air (7) associé de manière opérationnelle au dit trajet de flux de système circulant, dans lequel ledit collecteur d'air (7) est configuré pour enlever l'air dudit trajet de flux circulant pendant le fonctionnement.

26. Appareil de traitement thermique selon la revendication 25, dans lequel ledit élément collecteur d'air est un conteneur suspendu (7c) connectable au dit trajet de flux circulant.

27. Appareil de traitement thermique selon la revendication 25 ou 26, dans lequel ledit élément collecteur d'air est jetable.

28. Appareil de traitement thermique selon l'une quelconque des revendications 11 à 27, dans lequel ledit élément de chauffage tubulaire (3) et ledit cathéter (2) sont jetables.

29. Appareil de traitement thermique selon l'une quelconque des revendications 11 à 28, dans lequel ledit cathéter (2) a un corps cylindrique sensiblement allongé ayant une paroi externe s'étendant axialement avec une surface externe et des extrémités proximale et distale opposées et une ouverture centrale s'étendant à travers celle-ci, dans lequel, en position, ladite partie d'extrémité proximale est configurée pour pénétrer d'une distance dans la vessie d'un sujet,
ledit cathéter comprenant :
un port de drainage (32) situé sur la_ partie_ d'extrémité proximale de celui-ci ;
un ballon d'ancrage gonflable (21) adjacent au dit port de drainage ;
une partie de traitement thermique gonflable (22), qui, en pratique, est configurée pour transmettre un traitement thermique au sujet, et qui est dans une position intermédiaire entre ledit ballon d'ancrage (21) et ladite extrémité distale ; et
une pluralité de chambres d'isolation (30) positionnées dans une relation d'espacement circonférentiel vers l'intérieur de ladite paroi externe et s'étendant à partir d'une partie distale dudit cathéter à une distance souhaitée de celle-ci pour assurer l'isolation thermique ;
dans lequel ledit cathéter comprend un premier canal d'entrée de fluide en circulation (24a) et un deuxième canal de sortie de fluide en circulation (24b), chacun étant en communication fluide avec ladite partie de traitement thermique gonflable (22), et un troisième canal de drainage (33) s'étendant dudit port de drainage à une partie distale dudit cathéter pour fournir un trajet de flux d'écoulement urinaire à travers cela, et
dans lequel ledit élément de chauffage tubulaire (3) est en communication fluide avec ledit premier canal de fluide en circulation (24a) et est espacé dudit cathéter de telle sorte qu'il ne rentre pas dans la cavité du corps du sujet pendant l'utilisation.

30. Appareil de traitement thermique selon l'une quelconque des revendications 11 à 29, comprenant en outre un ensemble de capteur thermique (8) pour détecter la température du fluide en circulation dans le trajet de flux circulant dans le cathéter (2), ledit ensemble de capteur thermique (8) ayant deux extrémités opposées, l'une desdites extrémités pouvant être connectée au dit cathéter (2) et l'autre desdites extrémités pouvant être connectée au dit élément de chauffage tubulaire (3).

31. Appareil de traitement thermique selon les revendications 29 et 30, comprenant en outre un deuxième ensemble de capteur thermique (10) pour détecter la température du fluide circulant hors dudit cathéter (2), ledit deuxième ensemble de capteur thermique (10) ayant deux extrémités opposées, l'une desdites extrémités pouvant être connectée au dit cathéter et l'autre desdites extrémités pouvant être connectée à ladite pompe, ledit deuxième ensemble de capteur thermique (10) étant associé de manière opérationnelle au dit contrôleur (12).

32. Appareil de traitement thermique selon la revendication 31, comprenant en outre une première conduite (85) et une deuxième conduite (89) connectées à un canal respectif dudit premier canal de fluide en circulation (24a) dudit cathéter et dudit deuxième canal de fluide en circulation (24b) dudit cathéter, et le premier membre de capteur thermique tubulaire (84) et le deuxième membre de capteur thermique tubulaire (88) attachés à un canal respectif du premier canal de fluide (24a) et du deuxième canal de fluide (24b), dans lequel ledit cathéter (22), ledit élément thermique tubulaire (3), ladite première conduite (85) et ladite deuxième conduite (89), et ledit premier membre de capteur thermique tubulaire (84) et ledit deuxième membre de capteur thermique tubulaire (88) sont jetables après une seule utilisation.

33. Appareil de traitement thermique selon l'une quelconque des revendications 29 à 32, dans lequel ledit troisième canal de drainage (33) est configuré pour diriger un traitement médicamenteux à partir de ladite partie d'extrémité distale du cathéter pour s'évacuer au niveau dudit port de drainage.

34. Appareil de traitement thermique selon l'une quelconque des revendications 29 à 32, dans lequel ledit troisième canal de drainage (33) est configuré pour recevoir un membre de raidissement dans celui-ci.

35. Appareil de traitement thermique selon l'une quelconque des revendications 29 à 34, dans lequel ledit port de drainage urinaire (32) est situé à l'extrémité proximale du dit cathéter (2), et dans lequel ladite partie d'ancrage (21) est espacée dudit port de drainage urinaire (32) et située à une position intermédiaire entre ledit port de drainage (32) et ladite partie de traitement thermique gonflable (22) le long de la longueur dudit cathéter, et dans lequel chacun dudit port de drainage urinaire, de ladite partie d'ancrage, et de ladite partie de traitement thermique gonflable est configuré pour être en isolation fluide des autres.

36. Appareil de traitement thermique selon l'une quelconque des revendications 29 à 35, dans lequel ledit cathéter (2) est configuré pour pouvoir être inséré dans un urètre, dans lequel, en position, ledit ballon d'ancrage (21) est configuré pour se gonfler dans une forme bulbeuse pour ancrer ledit cathéter (2) dans un emplacement tel que ledit ballon d'ancrage réside dans la vessie pour toucher une partie du col de la vessie et maintenir ledit cathéter en position, et dans lequel ladite partie de traitement thermique gonflable est une partie d'ablation qui s'étend pour toucher de manière circonférentielle le tissu de la prostate pour procéder à l'ablation thermique du tissu de la prostate dans la cavité du corps.

37. Appareil de traitement thermique selon l'une quelconque des revendications 29 à 36, dans lequel ledit cathéter (2) est configuré pour diriger de l'air dans ledit ballon d'ancrage (21) pour le gonfler, pour diriger le fluide chauffé en circulation dans ladite partie de traitement thermique (22) pour l'expansion de celle-ci, et pour permettre à l'urine de s'écouler librement à partir dudit port de drainage (32) pendant le fonctionnement.

38. Appareil de traitement thermique selon l'une quelconque des revendications 29 à 37, dans lequel ledit cathéter (2) est configuré pour diriger le fluide chauffé dans ledit ballon d'ancrage (21) et dans ladite partie de traitement thermique gonflable (22) pour les gonfler.

39. Appareil de traitement thermique selon l'une quelconque des revendications 29 à 38, dans lequel ledit appareil de traitement thermique est configuré pour l'ablation de tissu, dans lequel ladite partie de traitement thermique gonflable (22) du cathéter est une partie d'ablation thermique, dans lequel ledit ballon d'ancrage (21) et ladite partie d'ablation thermique gonflable (22) sont en communication fluide, et dans lequel ledit cathéter est configuré pour faire circuler le fluide chauffé à travers ladite partie d'ablation thermique gonflable et ledit ballon d'ancrage.

40. Appareil de traitement thermique selon l'une quelconque des revendications 11 à 39, comprenant en outre un élément de boîtier de refroidissement adapté pour recevoir l'élément de chauffage tubulaire (3) dans un conteneur (14) contenant un bain d'un liquide de refroidissement (15), dans lequel ledit élément de chauffage tubulaire (3) est positionné de manière détachable dans le boîtier pour être en communication avec ledit liquide de refroidissement (15) et/ou pour chauffer et refroidir alternativement le fluide se déplaçant dans le trajet de flux circulant.
